# EUROPEAN PATENT SPECIFICATION

(11) **EP 4 240 191 B1**
(45) Date of publication and mention of the grant of the patent: **18.09.2024**
(21) Application number: 21805518.4
(22) Date of filing: 05.11.2021
(51) Int. Cl.: A24F 40/85, A61M 11/04, A61M 15/06, A24F 40/20, A24F 40/42

(54) **AEROSOL-GENERATING DEVICE WITH FLEXIBLE MEMBRANE COMPRISING AN OPENING**
AEROSOLERZEUGUNGSVORRICHTUNG MIT FLEXIBLER MEMBRAN MIT EINER ÖFFNUNG
DISPOSITIF DE GÉNÉRATION D'AÉROSOL DOTÉ D'UNE MEMBRANE FLEXIBLE COMPRENANT UNE OUVERTURE

(30) Priority: 05.11.2020 EP 20205872
(43) Date of publication of application: 13.09.2023
(73) Proprietor: Philip Morris Products S.A., 2000 Neuchâtel (CH)
(72) Inventor: YIM, Jun, Wei, 2000 Neuchâtel (CH); HOW, Jun, Jie, Singapore, 569873 (SG); DELA PAZ, Dennis, Yape, Singapore, 569873 (SG)
(74) Representative: Abitz & Partner
(86) International application number: PCT/EP2021/080728
(87) International publication number: WO 2022/096626

(56) References cited:
- EP-A1- 3 395 387
- WO-A1-2019/122878
- WO-A1-2019/242230
- CN-U- 207 940 343
- CN-U- 210 043 215
- US-A- 3 856 024
- US-A1- 2017 258 387

## Description

The present invention relates to an aerosol-generating device. The present invention further relates to a kit comprising the aerosol-generating device and a cleaning tool, and an aerosol-generating system comprising the aerosol-generating device, the cleaning tool and an aerosol-generating article. The present invention further relates to a method of cleaning the aerosol-generating device.

Aerosol-generating devices are known which heat but which do not burn aerosol-forming substrates in aerosol-generating articles such as tobacco. Such devices heat the aerosol-forming substrates to a sufficiently high temperature for generating an aerosol for inhalation by the user. These aerosol-generating devices normally include a cavity for receiving the aerosol-forming substrate. These devices are typically portable, hand-held devices and are required to be compact.

Residue or debris from aerosol-forming articles deposit in the cavity of the aerosol-generating devices over time. In some cases, such residues or debris may impair the function of these devices. Such residues or debris in the cavity may also affect the taste experienced by a user during aerosol-consumption and lead to clogging of the device. Cleaning the cavity of the aerosol-generating devices is often time-consuming and complicated, because the cavity in many such devices is not easily accessible from the outside. Aerosol-generating devices are known, which include openings with a door in order to access the cavity. These doors have to be opened and closed by a user increasing the time required for cleaning the aerosol-generating device. Such doors may be mechanical mechanisms involving springs or other biasing mechanisms. Such doors may break over prolonged usage. The efficiency of the cleaning procedure may be particularly low for any residues deposited at the upstream end, the bottom end of the cavity.

Aerosol-generating devices of the prior art are described in WO2019/122878A1, CN210043215U, CN207940343U, EP3395387A1, US3856024A and WO2019/242230A1. In particular, CN210043215U shows heat-not-burn smoking set which comprises a cleaning cover 7 arranged at an upstream end of a cigarette accommodation cavity.

It would be desirable to provide an aerosol-generating device which can easily be cleaned. It would furthermore be desirable to provide an aerosol-generating device, wherein debris or residue deposited in the cavity of the device can easily be removed. It would furthermore be desirable to provide an aerosol-generating device, whose cavity is easily accessible. It also would be desirable to provide an aerosol-generating device, allowing an easy cleaning of the upstream parts of the cavity for receiving the aerosol-generating article.

According to the present invention, there is provided an aerosol-generating device as described in the present claim 1.

According to a further embodiment of the present invention an aerosol-generating device comprising a cavity for receiving an aerosol-generating article comprising an aerosol-forming substrate is provided. A flexible membrane is arranged at the upstream end of the cavity. The flexible membrane comprises an opening which is in fluid communication with the cavity.

The opening in the flexible membrane may allow a cleaning tool to be easily pushed through. When applying pressure using the cleaning tool, the opening may widen due to the flexible membrane. This may allow the cleaning tool to pass through the opening. When pulling back the cleaning tool through the opening the opening may revert to its original shape and size due to the flexible nature of the membrane when the pressure of the cleaning tool ceases to be applied. The flexible membrane also may be rigid enough in order not to allow any residue from the outside to enter the cavity of the aerosol-generating device. The flexible membrane also may retain any residues in the cavity prior to cleaning.

The opening may have any geometrical shape useful for cleaning. The opening may have the shape of one or more of a slit, a cross, an incision, an aperture. All these geometrical shapes may expand when a cleaning tool is pushed through.

The opening may have a geometrical shape which enables fluid communication of the opening with the cavity. The opening in the flexible membrane may be adjacent to the cavity. The opening may not provide a fluid communication through the flexible membrane. In particular, the opening may be a slit or an incision formed in the flexible membrane, which does not provide fluid communication through the flexible membrane. In this case, an opening being large enough for the cleaning tool to pass through will only be formed upon application of a pressure to the opening of the flexible membrane.

The opening may be partially closed or completely closed in the absence of any outside pressure applied to the opening. This may enable the flexible membrane to prevent any debris from leaving the device without cleaning. The partially closed or completely closed opening may be opened upon application of pressure using for example a cleaning tool. The flexible membrane may enable the opening to be opened and widened, when pressure is applied on the flexible membrane.

The cavity may have a central longitudinal cavity axis. The opening may at least partly be arranged on the central longitudinal cavity axis.

This may ease the cleaning of the cavity using a cleaning tool, by pushing the cleaning tool through the opening. Since the opening may at least partly be arranged on the central longitudinal cavity axis, a long, elongate cleaning tool may particularly easy be pushed through the opening by either inserting the cleaning tool into the cavity along the central longitudinal cavity axis, applying force and pushing the cleaning tool through the opening or by pushing the cleaning tool from the upstream end of the cavity from outside the cavity through the opening into the cavity. The central longitudinal cavity axis also may enable centering the cleaning tool within the cavity along the central longitudinal cavity axis. This may avoid any damage to parts of the cavity done by the cleaning tool, when the cleaning tool brushes along the periphery of the cavity.

The aerosol-generating device may comprise a central longitudinal device axis. The central longitudinal device axis may be offset from the central longitudinal cavity axis. This may lead to the cavity being spaced apart from other components of the aerosol-generating device, such as electric circuitry or other electronic components of the aerosol-generating device. The cavity also may be spaced apart from the power source of the aerosol-generating device. This may ensure, that any air flow path through the cavity does not pass through the circuitry or other electronic components of the aerosol-generating device. Similarly, the air flow path may be directed around the power source of the aerosol-generating device. This may increase the lifetime of the aerosol-generating device. Alternatively, the central longitudinal cavity axis may coincide with the central longitudinal device axis.

The flexible membrane may comprise a concave part which bulges towards the interior of the cavity.

This may ensure that any debris formed in the cavity is not deposited in the center of the concave part of the flexible membrane. The debris may be deposited at the edges of the concave part which are not bulged towards the interior of the cavity or which are only bunched to minor extent. This may allow an easy cleaning of the device by pushing the cleaning tool through the opening.

The opening may be formed in the concave part of the flexible membrane. In particular, the opening may be formed in the concave part of the flexible membrane which protrudes the furthest into the interior of the cavity. This may ensure that any debris formed in the cavity is not deposited on the opening, but is deposited at the periphery of the concave part of the flexible membrane. This may ensure that the opening is not blocked by any debris.

The opening is configured to serve as an air inlet for the aerosol-generating device. The opening may be the most upstream part opening of the aerosol-generating device, allowing air to enter the aerosol-generating device. The opening may provide enough air to enter the cavity without significantly increasing the resistance to draw of the aerosol-generating device.

As used herein, the terms "upstream", and "downstream", are used to describe the relative positions of components, or portions of components, of the aerosol-generating device in relation to the direction in which air flows through the aerosol-generating device during use thereof along the airflow path. Aerosol-generating devices according to the invention comprise a proximal end through which, in use, an aerosol exits the device. The proximal end of the aerosol-generating device may also be referred to as the mouth end or the downstream end. The mouth end is downstream of the distal end. The mouth end may comprise a mouthpiece. The distal end of the aerosol-generating device may also be referred to as the upstream end. Components, or portions of components, of the aerosol-generating device may be described as being upstream or downstream of one another based on their relative positions with respect to the airflow path through the aerosol-generating device.

Because the opening serves as an air Inlet for the aerosol-generating device, it may be particularly advantageous if the opening is formed in the concave part of the flexible membrane. In particular, the opening may be a formed in the concave part protruding the furthest into the interior of the cavity. This may ensure that the opening, serving as an air inlet is not blocked by any debris. The opening may serve as the sole air inlet for the aerosol-generating device. Any debris may rather be collected at the periphery of the concave part, away from the opening.

The flexible membrane may comprise a convex part. The convex part may bulge outwards from the interior of the cavity. The convex part may be able to collect any debris formed in the cavity. The opening may be formed in the convex part of the flexible membrane. The opening may be formed in the convex part of the flexible membrane bulging outwards the furthest from the interior of the cavity. This may ensure that any debris formed in the cavity is collected near the opening. This may ease the cleaning of the device by inserting a cleaning tool through the opening and removing the debris being located near or at the opening. Locating the opening in a convex part of the flexible membrane may be particularly advantageous if additional air inlets are present in the aerosol-generating device. In this case, the opening may not serve as the sole air inlet, or as an air Inlet at all.

The flexible membrane may comprise a flat part. The entire flexible membrane may be formed as a flat part. This may still allow a user to insert a cleaning tool into the opening of the flexible membrane and cleaning the cavity by using the cleaning tool.

The flexible membrane may be part of a base of the cavity. This may ensure that the cavity can easily be cleaned by inserting the cleaning tool through the opening of the flexible membrane. The flexible membrane may also form the upstream end of the aerosol-generating device. This may ensure that any debris collected in the cavity on the flexible membrane can easily be removed through the opening out of the aerosol-generating device. The flexible membrane may be detachably attached to the aerosol-generating device. This may enable the flexible membrane to be replaced without the need for discarding the complete aerosol-generating device. The flexible membrane may be attached to one or both of sidewalls or to the base of the cavity of the aerosol-generating device.

The aerosol-generating device may further comprise a mounting element. The flexible membrane may be mounted in the mounting element. The mounting element may provide an easy connection between the flexible membrane and the remainder of the aerosol-generating device. The mounting element may be a mounting frame. This may allow an easy mounting of the flexible membrane within the mounting frame, wherein the mounting frame surrounds the flexible membrane. This may provide additional stability and structural integrity to the flexible membrane.

The flexible membrane may be detachably connected to the upstream end of the cavity. The flexible membrane may be detachably connected to the upstream end of the aerosol-generating device. In particular, the flexible membrane may be detachably connected to the mounting element. This may allow the replacement of the flexible membrane without the need to discard the complete aerosol-generating device.

The mounting element may comprise a rigid material. This may provide additional stability to the flexible membrane. The mounting element may comprise one or both of plastic and metal. The mounting frame may comprise one or more of polycarbonate (PC), a blend of polycarbonate and acrylonitrile butadiene styrene (PC/ABS), polypropylene, such as homo polypropylene, acrylonitril-butadien-styrene, polyamide, methyl polymethacrylate, polyethersulfone (PESU), or polyphenylsulfone (PPSU).

The aerosol-generating device may comprise a housing, the housing including the cavity. Preferably, the material of the mounting element comprises the same polymers as the material for the housing of the aerosol-generating device.

The opening comprises a slit. A slit may be particularly well suited to be opened by pushing through a cleaning tool. The slit may comprise a first and a second lip formed in the flexible membrane. The first and second lip in the flexible membrane may be easily pushed apart when pushing through the cleaning tool.

The first and second lip may abut each other. Abutting first and second lips may form an incision in the flexible membrane. Such an incision may not form an air Inlet of the aerosol-generating device. The incision may reliably cover the upstream end of the cavity if no pressure is applied to the incision. The incision may hold any debris and deposits formed during use of the aerosol-generating device in the cavity prior to cleaning. Upon using a cleaning tool and applying pressure to the incision, the cleaning tool may be pushed through the incision and any debris may reliably be removed from the cavity.

The opening may seal the cavity from upstream portions of the aerosol-generating device. This may prevent any debris deposited inside the cavity of the aerosol-generating device from leaving the device without any cleaning. The opening may seal the cavity from upstream portions of the aerosol-generating device. In particular incisions or slits may provide a sealing of the cavity.

In another embodiment the first and second lip of the slit may be spaced apart from each other. Thus, a slit may be formed, wherein a small opening is formed between the first and second lip of the slit. Such an opening may provide an air inlet for air to enter the cavity of the aerosol-generating device. The opening furthermore may facilitate an easy opening of the slit when a cleaning tool is pushed through the slit. The width of the slits may be between 0.05 to 0.3 mm, preferably between 0.1 to 0.2 mm. These widths may on the one hand provide some access for air into the device, but may on the other hand prevent any debris from the cavity of the aerosol-generating device escaping the cavity prior to cleaning.

At least two openings are formed in the flexible membrane. The at least two openings may provide different positions in the flexible membrane for any cleaning tool to pass through the membrane. The at least two openings comprise slits which intersect. The intersecting slits may be shaped as a cross, or may have a T-shape or any other suitable shape. It may be particularly easy for a cleaning tool to pass through intersecting slits in the flexible membrane during any cleaning procedure.

The at least two intersecting slits form a central portion, where both intersecting slits are crossing each other. The central portion may particularly easy be pushed through by any cleaning tool passing through the flexible membrane. The central portion may comprise an aperture. The aperture may provide an air inlet for air passing from the outside into the cavity of the aerosol-generating device. The width of the aperture may be larger than the width of the slits if the slits comprise two opposing first and second lips, which are spaced apart from each other. The diameter of the aperture may be between 0.4 mm to 1.5 mm, preferably between 0.5 mm to 1 mm. This configuration of an aperture with the slits comprising opposing first and second lips being spaced apart from each other can easily be pushed through by a cleaning tool. This configuration also may provide additional openings for air passing from the outside into the cavity of the aerosol-generating device.

A plurality of slits intersecting in a central portion of the opening may separate a plurality of flexible regions in the membrane which may bend under the pressure applied by the cleaning tool. For example, three slits extending from a central portion may define three flexible regions. Similarly, four slits extending from a central portion may lead to four flexible regions within the flexible membrane which can be blended under pressure from the cleaning tool to allow the cleaning tool to pass through the opening.

The flexible membrane may comprise an elastomeric material. Elastomeric materials are particularly flexible and are able to return to their original shape and size after any force applied by a cleaning tool has ceased to be applied. The elastomeric material may comprise one or more of: silicon, polyester elastomer and polyurethane elastomer. The elastomer may be a thermoset or thermoplastic elastomer, preferably a thermoplastic elastomer.

The aerosol-generating device may furthermore comprise a heating element. The heating element may serve to heat an aerosol-generating article received in the cavity in order to produce an aerosol. The heating element may comprise one or both of an inductive heating element and a resistive heating element. The inductive heating element may comprise an inductor coil disposed around at least a portion of the cavity and connected to a power supply. The power supply may be configured to provide an alternating electric current to the inductor coil, such that in use, the inductor coil may generate an alternating magnetic field to heat a susceptor by creating eddy currents. The susceptor may be part of one or both of the aerosol-generating device and the aerosol-generating article received in the cavity of the aerosol-generating device. Preferably, the susceptor may be part of the aerosol-generating device or the aerosol-generating article.

The aerosol-generating device may comprise a downstream end comprising an opening for receiving an aerosol-generating article in the cavity. The cavity of the aerosol-generating article may have an elongate shape. This may allow the cavity to receive tubular or rod-shaped aerosol-generating articles. The flexible membrane at the upstream end of the cavity may have an elongated, flat shape. A flexible membrane with such a shape may particularly well suited to cover the upstream end of the cavity. Such a membrane also might ease the handling of the aerosol-generating device by allowing a user to handle the aerosol-generating device by holding the device by the flexible membrane. The flexible membrane may provide a component having a rough surface allowing the user to hold the aerosol generating device by the flexible membrane without risk of the aerosol-generating device slipping out of the hands of the user. The flexible membrane may provide additional space for further components of the aerosol-generating device, such as USB ports and other connection ports.

The aerosol-generating device may further comprise electric circuitry. The electric circuitry may comprise a microprocessor, which may be a programmable microprocessor. The microprocessor may be part of a controller. The electric circuitry may comprise further electronic components. The electric circuitry may be configured to regulate a supply of power to the heating element, particularly to the induction coil or the resistive heating element. Power may be supplied to the heating element continuously following activation of the aerosol-generating device or may be supplied intermittently, such as on a puff-by-puff basis. The power may be supplied to the heating element in the form of pulses of electrical current. The electric circuitry may be configured to monitor the electrical resistance of the heating element, and preferably to control the supply of power to the heating element dependent on the electrical resistance of the heating element.

The aerosol-generating device may further comprise a power supply, typically a battery, within a main body of the aerosol-generating device. In one embodiment, the power supply is a Lithium-ion battery. Alternatively, the power supply may be a Nickel-metal hydride battery, a Nickel cadmium battery, or a Lithium based battery, for example a Lithium-Cobalt, a Lithium-Iron-Phosphate, Lithium Titanate or a Lithium-Polymer battery. As an alternative, the power supply may be another form of charge storage device such as a capacitor. The power supply may require recharging and may have a capacity that enables to store enough energy for one or more usage experiences; for example, the power supply may have sufficient capacity to continuously generate aerosol for a period of around six minutes or for a period of a multiple of six minutes. In another example, the power supply may have sufficient capacity to provide a predetermined number of puffs or discrete activations of the heating element.

The invention also provides a kit comprising an aerosol-generating device as described herein and a cleaning tool for cleaning the cavity of the aerosol-generating device. The cleaning tool may have an elongated shape. The elongated shape of the cleaning tool may correspond to the elongated shape of the cavity. This may facilitate easy cleaning of the cavity by inserting the cleaning tool into the cavity of the aerosol-generating device.

The cleaning tool may comprise a cleaning head. The cleaning head may be configured to open the opening when pressure is applied with the cleaning tool. The cleaning head may be configured to be pushed through the opening of the flexible membrane. The cleaning tool may comprise a central longitudinal section comprising one or both of a metal or a plastic. The metal may be stiff enough in order to enable a user to push the cleaning tool through the opening of the flexible membrane. The cleaning tool may comprise a central longitudinal stick from which bristles protrude. The longitudinal stick may comprise one or more of metal, plastics or wood. The bristles may be configured for cleaning the cavity. The cavity may comprise sidewalls and the bristles may be configured for cleaning the sidewalls of the cavity. The cleaning head of the cleaning tool may comprise absorbent material, configured for absorbing any residues located in the cavity. The cleaning head may comprise cotton as an absorbent material.

A cross-sectional with of the cleaning head may be equal to or smaller than a width of the opening. A cross-sectional width of the cleaning head may be equal to or smaller than a length of a slit formed in the flexible membrane. This may enable the cleaning head to be easily passed through either the opening or the slit.

The invention also provides an aerosol-generating system, which may comprise an aerosol-generating device as described herein, an aerosol-generating article for being received in the cavity and a cleaning tool for cleaning the cavity. The aerosol-generating article may comprise aerosol-forming substrate.

As used herein, the term 'aerosol-forming substrate' relates to a substrate capable of releasing one or more volatile compounds that can form an aerosol. Such volatile compounds may be released by heating the aerosol-forming substrate. An aerosol-forming substrate may conveniently be part of an aerosol-generating article or smoking article.

The aerosol-forming substrate may be a solid aerosol-forming substrate. The aerosol-forming substrate may comprise both solid and liquid components. The aerosol-forming substrate may comprise a tobacco-containing material containing volatile tobacco flavour compounds which are released from the substrate upon heating. The aerosol-forming substrate may comprise a non-tobacco material. The aerosol-forming substrate may comprise an aerosol former that facilitates the formation of a dense and stable aerosol. Suitable aerosol formers are well known in the art and include, but are not limited to: polyhydric alcohols such as, for example, triethylene glycol, 1,3-butanediol, propylene glycol and glycerine; esters of polyhydric alcohols such as, for example, glycerol mono-, di- or triacetate; aliphatic esters of mono-, di- or polycarboxylic acids such as, for example, dimethyl dodecanedioate and dimethyl tetradecanedioate; and combinations thereof.

The aerosol former may comprise one or more of glycerine and propylene glycol. The aerosol former may consist of glycerine or propylene glycol or of a combination of glycerine and propylene glycol. Preferred examples of suitable aerosol formers are glycerine and propylene glycol.

Preferably, the amount of aerosol former is between 6 percent and 20 percent by weight on a dry weight basis of the aerosol-forming substrate, more preferably, the amount of aerosol former is between 8 percent and 18 percent by weight on a dry weight basis of the aerosol-forming substrate, most preferably the amount of aerosol former is between 10 percent and 15 percent by weight on a dry weight basis of the aerosol-forming substrate. For some embodiments the amount of aerosol former has a target value of about 13 percent by weight on a dry weight basis of the aerosol-forming substrate. The most efficient amount of aerosol former will depend also on the aerosol-forming substrate, whether the aerosol-forming substrate comprises plant lamina or homogenized plant material. For example, among other factors, the type of substrate will determine to which extent the aerosol-former can facilitate the release of substances from the aerosol-forming substrate.

For these reasons, the aerosol-forming substrate of the present invention is capable of efficiently generating sufficient amount of aerosol at relatively low temperatures. A temperature of between 150 degrees Celsius and 200 degrees Celsius in the heating chamber is sufficient for the aerosol-forming substrate of the present invention to generate sufficient amounts of aerosol while in aerosol-generating devices using tobacco cast leave sheets typically temperatures of about 250 degrees Celsius are employed.

Preferably, the aerosol-forming substrate comprises cut-filler. In this document, "cut-filler" is used to refer to a blend of shredded plant material, in particular leaf lamina, processed stems and ribs, homogenized plant material, like for example made into sheet form using casting or papermaking processes. The cut filler may also comprise other after-cut, filler tobacco or casing. According to preferred embodiments of the invention, the cut-filler comprises at least 25 percent of plant leaf lamina, more preferably, at least 50 percent of plant leaf lamina, still more preferably at least 75 percent of plant leaf lamina and most preferably at least 90 percent of plant leaf lamina. Preferably, the plant material is one of tobacco, mint, tea and cloves, however, the invention is equally applicable to other plant material that has the ability to release substances upon the application of heat that can subsequently form an aerosol.

Preferably, the tobacco plant material comprises lamina of one or more of bright tobacco lamina, dark tobacco, aromatic tobacco and filler tobacco. Bright tobaccos are tobaccos with a generally large, light coloured leaves. Throughout the specification, the term "bright tobacco" is used for tobaccos that have been flue cured. Examples for bright tobaccos are Chinese Flue-Cured, Flue-Cured Brazil, US Flue-Cured such as Virginia tobacco, Indian Flue-Cured, Flue-Cured from Tanzania or other African Flue Cured. Bright tobacco is characterized by a high sugar to nitrogen ratio. From a sensorial perspective, bright tobacco is a tobacco type which, after curing, is associated with a spicy and lively sensation. According to the invention, bright tobaccos are tobaccos with a content of reducing sugars of between about 2.5 percent and about 20 percent of dry weight base of the leaf and a total ammonia content of less than about 0.12 percent of dry weight base of the leaf. Reducing sugars comprise for example glucose or fructose. Total ammonia comprises for example ammonia and ammonia salts. Dark tobaccos are tobaccos with a generally large, dark coloured leaves. Throughout the specification, the term "dark tobacco" is used for tobaccos that have been air cured. Additionally, dark tobaccos may be fermented. Tobaccos that are used mainly for chewing, snuff, cigar, and pipe blends are also included in this category. Typically, these dark tobaccos are air cured and possibly fermented. From a sensorial perspective, dark tobacco is a tobacco type which, after curing, is associated with a smoky, dark cigar type sensation. Dark tobacco is characterized by a low sugar to nitrogen ratio. Examples for dark tobacco are Burley Malawi or other African Burley, Dark Cured Brazil Galpao, Sun Cured or Air Cured Indonesian Kasturi. According to the invention, dark tobaccos are tobaccos with a content of reducing sugars of less than about 5 percent of dry weight base of the leaf and a total ammonia content of up to about 0.5 percent of dry weight base of the leaf. Aromatic tobaccos are tobaccos that often have small, light coloured leaves. Throughout the specification, the term "aromatic tobacco" is used for other tobaccos that have a high aromatic content, e.g. of essential oils. From a sensorial perspective, aromatic tobacco is a tobacco type which, after curing, is associated with spicy and aromatic sensation. Example for aromatic tobaccos are Greek Oriental, Oriental Turkey, semi-oriental tobacco but also Fire Cured, US Burley, such as Perique, Rustica, US Burley or Meriland. Filler tobacco is not a specific tobacco type, but it includes tobacco types which are mostly used to complement the other tobacco types used in the blend and do not bring a specific characteristic aroma direction to the final product. Examples for filler tobaccos are stems, midrib or stalks of other tobacco types. A specific example may be flue cured stems of Flue Cure Brazil lower stalk.

The cut-filler suitable to be used with the present invention generally may resemble to cut-filler used for conventional smoking articles. The cut width of the cut filler preferably is between 0.3 millimeters and 2.0 millimeters, more preferably, the cut width of the cut filler is between 0.5 millimeters and 1.2 millimeters and most preferably, the cut width of the cut filler is between 0.6 millimeters and 0.9 millimeters. The cut width may play a role in the distribution of heat inside the substrate portion of the article. Also, the cut width may play a role in the resistance to draw of the article. Further, the cut width may impact the overall density of the substrate portion.

The strand length of the cut-filler is to some extent a random value as the length of the strands will depend on the overall size of the object that the strand is cut off from. Nevertheless, by conditioning the material before cutting, for example by controlling the moisture content and the overall subtlety of the material, longer strands can be cut. Preferably, the strands have a length of between about 10 millimeters and about 40 millimeters before the strands are formed into the substrate section. Obviously, if the strands are arranged in a substrate section in a longitudinal extension where the longitudinal extension of the section is below 40 millimeters, the final substrate section may comprise strands that are on average shorter than the initial strand length. Preferably, the strand length of the cut-filler is such that between about 20 percent and 60 percent of the strands extend along the full length of the substrate portion. This prevents the strands from dislodging easily from the substrate section.

As used herein, the term 'aerosol-generating article' refers to an article comprising the aerosol-forming substrate that is capable of releasing volatile compounds that can form an aerosol. For example, an aerosol-generating article may be a smoking article that generates an aerosol that is directly inhalable into a user's lungs through the user's mouth. An aerosol-generating article may be disposable. The aerosol-generating article may comprise a substrate portion, which comprises the aerosol-forming substrate. The substrate portion may have a length of between 20 millimeters and 60 millimeters, preferably a length from 12 millimeters to 16 millimeters. The diameter of the aerosol-generating article may be from 12 millimeters to 16 millimeters, preferably between around 6.5 millimeters and 7.5 millimeters, more preferably between around 6.5 millimeters and 7.3 millimeters.

The aerosol-generating article may be substantially cylindrical in shape. The aerosol-generating article may be substantially elongate. The aerosol-generating article may have a length and a circumference substantially perpendicular to the length. The aerosol-generating article may be substantially rod shaped. The aerosol-forming substrate may be substantially cylindrical in shape. The aerosol-forming substrate may be substantially elongate. The aerosol-forming substrate may also have a length and a circumference substantially perpendicular to the length. The aerosol-forming substrate may be substantially rod shaped.

Apart from the aerosol-forming substrate, the aerosol-generating article may furthermore comprise one or more of: a support element located downstream of the aerosol-forming substrate, an aerosol-cooling element located downstream of the support element, and an outer wrapper circumscribing the aerosol-forming substrate. The support element and the aerosol-cooling element may be in a linear sequential arrangement together with the aerosol-forming substrate. The support element may abut the aerosol-forming substrate.

Also provided is a method for cleaning the aerosol-generating device as described herein. The method may comprise one or both of:
- inserting a cleaning tool into the cavity through the downstream end of the cavity and pushing the cleaning tool through the opening and removing the cleaning tool from the cavity, or
- pushing a cleaning tool through the opening of the flexible membrane into the cavity from the upstream end of the aerosol-generating device and removing the cleaning tool from the cavity.

Pushing the cleaning tool through the opening of the flexible membrane may allow the removal of debris from the cavity. No further manual steps are required by the user apart from handling the cleaning tool. For example, the method does not require the user opening a door in the upstream part of the aerosol-generating device in order to allow the cleaning tool to pass through. Due to the opening provided in flexible membrane, any debris nevertheless can easily be removed from the cavity when the opening is widened upon applying a pressure with the cleaning tool.

Removing the cleaning tool from the cavity may comprise pulling the cleaning tool back through the opening of the flexible membrane out of the cavity. The opening thereafter may return to its original shape and size due to the flexibility of the membrane. After cleaning, the aerosol-generating device may be re-used. When re-using the device, the flexible membrane may reliably retain any debris deposited in the cavity within the aerosol-generating device.

The aerosol-generating device may comprise a bottom end face located at the upstream end of the device. The flexible membrane may provide an upstream bottom cover for the aerosol-generating device. The upstream bottom cover may cover at least parts of the bottom end face. The flexible membrane may span an area corresponding e.g. in shape and size to a cross-sectional area of the cavity. The flexible membrane may cover an area comprising the opening. The flexible membrane may comprise the opening and an area surrounding the opening. The flexible membrane may span a larger area than the opening. The flexible membrane may span the entire bottom end face of the device. The flexible membrane may span at least around 25 percent, at least around 50 percent, at least around 75%, at least around 80 percent of the bottom end face of the aerosol-generating device.

The disclosure also provides an aerosol-generating device which may comprise a cavity for receiving an aerosol-generating article comprising an aerosol-forming substrate. The cavity may have a central longitudinal axis. A flexible membrane may be arranged at the upstream end of the cavity. The flexible membrane may comprise an incision. This incision may at least partly be arranged on the central longitudinal axis of the cavity.

The disclosure furthermore provides an aerosol-generating device comprising a cavity for receiving an aerosol-generating article comprising an aerosol-forming substrate. The cavity has a central longitudinal axis. A flexible membrane is arranged at the upstream end of the cavity. The flexible membrane comprises an incision. The incision is at least partly arranged on the central longitudinal axis of the cavity.

The incision may be opened upon application of a pressure to the flexible membrane when pushing the cleaning tool through the membrane. Since the incision may at least partly be arranged on the central longitudinal axis of the cavity, a cleaning tool can particularly easily be inserted into the cavity and pushed through the incision.

After having pulled the cleaning tool back through the opening, the incision may return to its original shape and size due to the flexibility of the membrane.

The incision may have a lined shape. This may allow a cleaning tool to be easily pushed through the incision. The incision may comprise a slit. The slit may comprise a first and a second lip formed in the flexible membrane. The first and second lip may abut each other or may be spaced apart from each other.

If the incision comprises a first and the second lip abutting each other, the incision may not serve as an air inlet. In this case separate air inlets may be present in the aerosol-generating device which allow air from the outside to enter the cavity.

The first and second lip which may be spaced apart from each other and may form a slit as already described herein. There may be more than one slit, which also may intersect as described herein.

Features described in relation to one embodiment may equally be applied to other embodiments of the invention.

The invention will be further described, by way of example only, with reference to the accompanying drawings in which:
Fig. 1A and 1B show cross-sectional views of an aerosol-generating device according to one embodiment of the invention with a flexible membrane before and during the cleaning procedure;
Fig. 2A and 2B show cross-sectional views of another aerosol-generating device of the invention with a flexible membrane before and during a cleaning procedure;
Fig. 3 shows a perspective view of an aerosol-generating device showing the upstream end of the aerosol-generating device with the flexible membrane;
Fig. 4 depicts a schematic view of a flexible membrane including slits as one embodiment of the opening;
Fig. 5A and 5B depict different slits formed in the flexible membrane; and
Fig. 6A and 6B depict different incisions formed in the flexible membrane.

In the following the same elements are marked with the same reference numerals throughout all the figures.

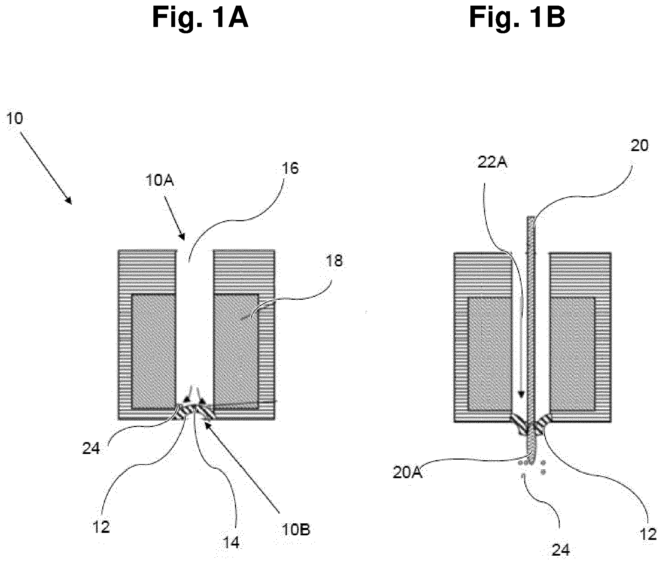

Fig. 1A shows a cross-sectional view of an aerosol-generating device 10. The aerosol-generating device comprises a cavity 16 for receiving an aerosol-generating article. The cavity 16 is surrounded by a heating element 18, which can be a resistive or inductive heating element. The device 10 comprises a downstream end 10A, through which an aerosol-generating article can be received in the cavity 16. At the upstream end 10B of the aerosol-generating device 10 a flexible membrane 12 is present, which includes an opening 14. The flexible membrane 12 comprises a concave part bulging towards the interior of the cavity of the aerosol-generating device 10. This leads to any debris 24 deposited in the cavity 16 to accumulate at the periphery of the concave part and not at or around the opening 14 (see arrows in Fig. 1A). This is advantageous, if the opening 14 also serves as an air inlet for the cavity 16 of the aerosol-generating device 10. In this case, the concave part of the membrane can prevent any debris from blocking the opening.

Fig. 1B shows a cross-sectional view of the aerosol-generating device 10 of Fig. 1A, wherein a cleaning tool 20 is inserted into the cavity 16 of the device from the downstream end 10A (see arrow 22A indicating the direction of insertion of the cleaning tool into the cavity). The cleaning tool 20 also can be inserted into the cavity by pushing the cleaning head through the opening 14 from the upstream end 10B of the device into the cavity 16 in a direction opposite to the direction indicated by the arrow 22A. Cleaning head 20A of the cleaning tool 20 is pushed through the opening 14 and the flexible membrane 12 is opening in order to allow the passage of the cleaning tool through the opening. Debris 24 deposited in the cavity 16 is thereby removed from the cavity. After retracting the cleaning tool 20 through the opening 14 of the flexible membrane 12, the opening and the flexible membrane return to their original shape and dimensions as shown in Fig. 1A. The cleaned aerosol-generating device 10 can then be re-used by a user for inhaling an aerosol.

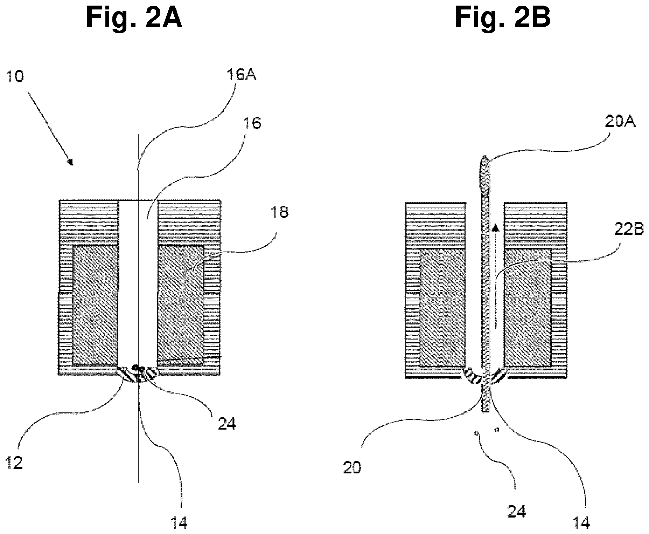

Fig. 2A and 2B depict an aerosol-generating device 10, which is similar to the device shown in the Fig. 1A and 1B. The difference is, that in the device of Fig. 2A the flexible membrane 12 comprises a convex part. The convex part bulges outwards from the interior of the cavity and the opening 14 is located in the convex part bulging outwards the furthest from the interior of the cavity. This results in the debris 24 deposited in the cavity accumulating on or near the opening 14. This is particularly advantageous, if other air inlets are present in the aerosol-generating device 10, so that the opening 14 does not have to serve as an air inlet. If the debris 24 accumulates near the opening 14, it can easily be removed by inserting the cleaning tool into the opening. As shown in Fig. 2B a cleaning tool 20 can be inserted into the cavity along the central longitudinal axis 16A of the cavity by pushing the cleaning head 20A through the opening 14 in the flexible membrane 12 from the upstream end of the device (direction of insertion of the cleaning tool 20 into the cavity 16 indicated by the arrow 22B). This cleaning procedure may also serve to remove any debris 24 deposited in the cavity 16 out of the aerosol-generating device 10. The cleaning tool 20 can then be retracted out of the cavity 16 by pulling cleaning tool through the opening 14 from the upstream end of the aerosol-generating device 10.

The cleaning procedures depicted in the Fig. 1B and 2B only require a user to handle the cleaning tool 20, but do not require additional handling steps, such as the opening of a door in the upstream part of the cavity. Due to the presence of the flexible membrane no complicated mechanical mechanisms involving springs or other biasing mechanisms need to be present in order to facilitate the opening of the bottom part of the device. Furthermore, the flexible membrane 12 with the opening 14 can ensure that any debris formed in the cavity does not leave the cavity prior to cleaning. This also facilitates the handling of the aerosol-generating device and avoids any undesired spill-over of the debris in the absence of a cleaning procedure.

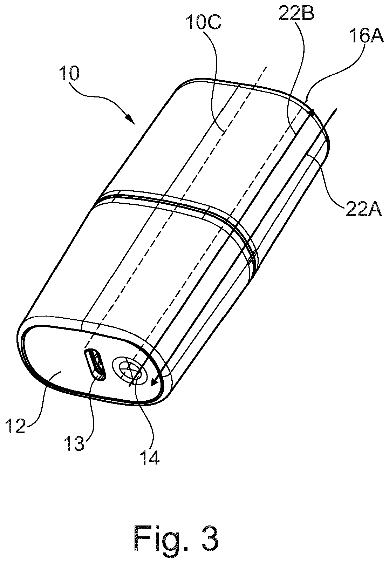

Fig. 3 depicts a perspective view of an aerosol-generating device 10. The upstream end of the device can clearly be seen. It includes the flexible membrane 12 and the opening 14 comprising two intersecting slits. Additionally, a USB port 13 is present which can be used in order to recharge the power source present in the aerosol-generating device 10. Two arrows depicted with 22B or 22A indicate possible directions of insertion of the cleaning tool into the cavity (not shown) of the device. The arrow denoted with 22B shows insertion of the cleaning tool from the upstream end of the device through the opening 14 into the cavity. The arrow denoted with 22A shows the insertion of the cleaning tool in opposite direction from the cavity through the opening 14. The possible directions of insertion of the cleaning tool are along the central longitudinal cavity axis 16A. This central longitudinal cavity axis 16A is offset from the central longitudinal device axis 10C. This allows the air flow path through the cavity to be directed spaced apart from the other internal components of the aerosol-generating device, such as circuitry or a power source.

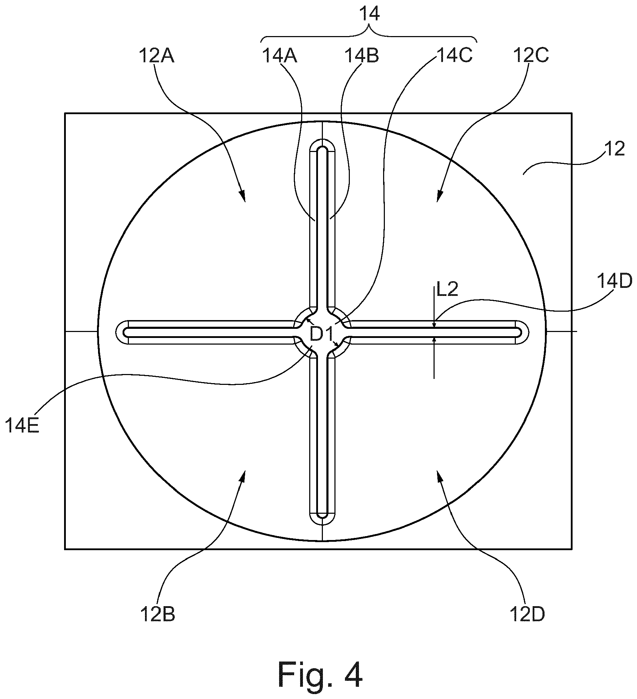

Fig. 4 depicts a top view of an embodiment of a flexible membrane 12, which comprises slits 14. The slit 14 comprises a first lip 14A and an opposing second lip 14B which are spaced apart from each other. Both spaced apart lips form a slit with a small opening having the width L2, denoted with the reference numeral 14D. Two slits 14 are present which intersect in the central portion and form an aperture 14C in the central portion. The aperture has a width D1 denoted with the reference numeral 14E, with D1 being larger than the width L2. Thus, cross-shaped slits are formed. These slits, in particular the aperture 14C may serve as an air inlet for the aerosol-generating device. These slits also may be easy to open by pushing a cleaning tool therethrough. In particular, four different flexible sections 12A, 12B, 12C and 12D of the flexible membrane 12 are formed which can easily be bended when applying pressure using a cleaning tool.

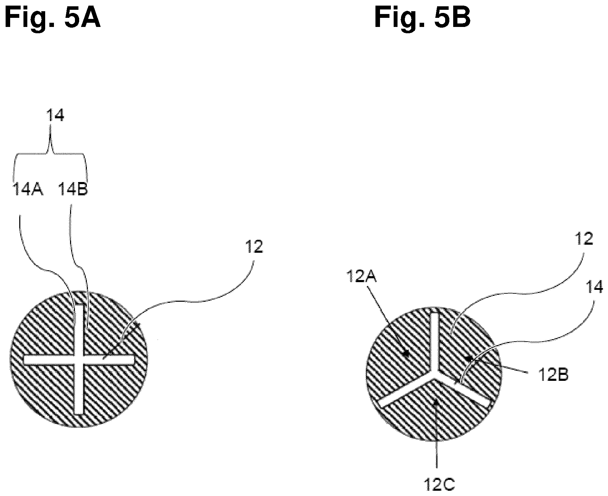

Fig. 5A and 5C show top views of two flexible membranes 12, which both comprise different slits 14. Fig. 5A shows two slits 14 intersecting and forming a cross. The slits 14 comprise two opposing first lips 14A and a second lip 14B, which are spaced apart from each other, thereby forming a small opening. Fig. 5B shows slits 14 in the membrane 16, which form a 3-ray star, so that three separate flexible sections 12A, 12B and 12C are formed in the flexible membrane 12. These three flexible sections can easily be pushed away when passing a cleaning tool through the flexible membrane.

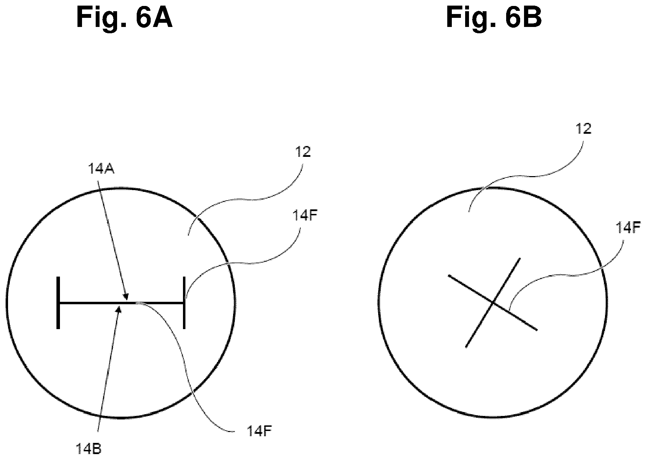

Fig. 6A and 6B depict flexible membranes 12 including incisions 14F. These incisions 14F comprise a first lip 14A and a second lip 14B which directly about each other and which therefore do not form a narrow slit-like opening in the membrane 12. These incisions therefore increase the resistance to draw of the aerosol-generating device and do not serve as air inlets. In this case, additional air inlets are normally present in the aerosol-generating device allowing the entrance of air into the cavity of the device. Nevertheless, these incisions 14F allow a cleaning tool to be pushed through the flexible membrane 12 thereby enabling an easy cleaning of the cavity of the aerosol-generating device.

## Claims

1. Aerosol-generating device (10) comprising:
a cavity (16) for receiving an aerosol-generating article comprising an 'aerosol-forming substrate, wherein the 'aerosol-forming substrate is a solid 'aerosol-forming substrate, and
a flexible membrane (12) arranged at the upstream end of the cavity (16), the flexible membrane (12) comprising an opening (14) in fluid communication with the cavity (16), wherein the flexible membrane (14) is provided at the upstream end (10B) of the aerosol-generating device (10), wherein the opening (14) is configured to serve as an air inlet for the aerosol-generating device (10),
wherein at least two openings (14) are formed in the flexible membrane (12), the at least two openings comprising slits (14) which intersect, and wherein the at least two intersecting slits (14) form a central portion (14C).

2. The aerosol-generating device (10) of the preceding claim, wherein the cavity (16) has a central longitudinal cavity axis (16A) and wherein the opening (14) is is at least partly arranged on the central longitudinal cavity axis (16A).

3. The aerosol-generating device (10) of the preceding claim, wherein the aerosol-generating device (10) comprises a central longitudinal device axis (16C) and wherein the central longitudinal device axis (10C) is offset from the central longitudinal cavity axis (16A).

4. The aerosol-generating device (10) of any of the preceding claims, wherein the flexible membrane (12) comprises a concave part which bulges towards the interior of the cavity (16), preferably wherein the opening (14) is formed in the concave part of the flexible membrane (12).

5. The aerosol-generating device (10) of any of the preceding claims, wherein the opening (14) is configured to serve as the sole air inlet for the aerosol-generating device (10).

6. The aerosol-generating device (10) of any of the claims 1 or 3, wherein the flexible membrane (12) comprises a convex part which bulges outwards from the interior of the cavity (16), preferably wherein the opening (14) is formed in the convex part of the flexible membrane (12).

7. The aerosol-generating device (10) of any of the preceding claims, further comprising a mounting element, wherein the flexible membrane (12) is mounted in the mounting element, preferably wherein the mounting element is arranged at the upstream end of the aerosol-generating device (10), most preferably wherein the mounting element is a mounting frame.

8. The aerosol-generating device (10) of device any of the preceding claims, wherein the opening (14) comprises a slit, optionally wherein the slit comprises a first and a second lip formed in the flexible membrane (12), the first and second lip abutting each other.

9. The aerosol-generating device (10) of any of the preceding claims, wherein the central portion (14C) comprises an aperture, preferably wherein the width of the aperture is larger than the width of the slits.

10. A kit comprising an aerosol-generating device (10) of any of the preceding claims and a cleaning tool (20) for cleaning the cavity (16) of the aerosol-generating device (10).

11. The kit of the claim 10, wherein the cleaning tool (20) comprises a cleaning head (20A), the cleaning head (20A) being configured to open the opening (14) when pressure is applied with the cleaning tool (20), preferably wherein the cleaning head (20A) is configured to be pushed through the opening (14) of the flexible membrane (12).

12. Aerosol-generating system, comprising an aerosol-generating device (10) of any of the claims 1 to 9, an aerosol-generating article for being received in the cavity (16), and a cleaning tool (20) for cleaning the cavity (16).

13. A method of cleaning the aerosol-generating device (10) of any of the claims 1 to 9, the aerosol-generating device (10) comprising a downstream end (10A) for receiving the aerosol-generating article in the cavity (16), the method comprising one or both of:
- inserting a cleaning tool (20) into the cavity (16) through the downstream end (10A) and pushing the cleaning tool (20) through the opening (14) and removing the cleaning tool (20) from the cavity (16), or
- pushing a cleaning tool (20) through the opening (14) of the flexible membrane (12), into the cavity (16) from the upstream end (10B) of the aerosol-generating device (10); and removing the cleaning tool (20) from the cavity (16).

14. The method of cleaning according to the preceding claim, wherein removing the cleaning tool (20) from the cavity (16) comprises:
- pulling the cleaning tool (20) back through the opening (14) of the flexible membrane (12) out of the cavity (16).

## Patentansprüche

1. Aerosolerzeugungsvorrichtung (10), umfassend:
einen Hohlraum (16) zum Aufnehmen eines aerosolerzeugenden Artikels, umfassend ein aerosolerzeugendes Substrat, wobei das aerosolerzeugende Substrat ein festes aerosolerzeugendes Substrat ist, und
eine flexible Membran (12), die an dem vorgelagerten Ende des Hohlraums (16) angeordnet ist, wobei die flexible Membran (12) eine Öffnung (14) in Fluidverbindung mit dem Hohlraum (16) umfasst, wobei die flexible Membran (14) an dem vorgelagerten Ende (10B) der Aerosolerzeugungsvorrichtung (10) vorgesehen ist, wobei die Öffnung (14) ausgelegt ist, als ein Lufteinlass für die Aerosolerzeugungsvorrichtung (10) zu dienen,
wobei wenigstens zwei Öffnungen (14) in der flexiblen Membran (12) gebildet sind, die wenigstens zwei Öffnungen sich kreuzende Schlitze (14) umfassen, und wobei die wenigstens zwei sich kreuzenden Schlitze (14) einen zentralen Abschnitt (14C) bilden.

2. Aerosolerzeugungsvorrichtung (10) nach dem vorhergehenden Anspruch, wobei der Hohlraum (16) eine zentrale Hohlraumlängsachse (16A) aufweist und wobei die Öffnung (14) wenigstens teilweise auf der zentralen Hohlraumlängsachse (16A) angeordnet ist.

3. Aerosolerzeugungsvorrichtung (10) nach dem vorhergehenden Anspruch, wobei die Aerosolerzeugungsvorrichtung (10) eine zentrale Vorrichtungslängsachse (16C) umfasst und wobei die zentrale Vorrichtungslängsachse (10C) von der zentralen Hohlraumlängsachse (16A) versetzt ist.

4. Aerosolerzeugungsvorrichtung (10) nach einem der beliebige vorhergehenden Ansprüche, wobei die flexible Membran (12) einen konkaven Teil aufweist, der sich in Richtung des Inneren des Hohlraums (16) wölbt, wobei die Öffnung (14) bevorzugt in dem konkaven Teil der flexiblen Membran (12) ausgebildet ist.

5. Aerosolerzeugungsvorrichtung (10) nach einem der vorhergehenden Ansprüche, wobei die Öffnung (14) ausgelegt ist, als einziger Lufteinlass für die Aerosolerzeugungsvorrichtung (10) zu dienen.

6. Aerosolerzeugungsvorrichtung (10) nach einem der Ansprüche 1 oder 3, wobei die flexible Membran (12) einen konvexen Teil umfasst, der sich von dem Inneren des Hohlraums (16) nach außen wölbt, wobei die Öffnung (14) bevorzugt in dem konvexen Teil der flexiblen Membran (12) gebildet ist.

7. Aerosolerzeugungsvorrichtung (10) nach einem beliebigen der vorhergehenden Ansprüche, ferner umfassend ein Befestigungselement, wobei die flexible Membran (12) in dem Befestigungselement befestigt ist, bevorzugt wobei das Befestigungselement an dem vorgelagerten Ende der Aerosolerzeugungsvorrichtung (10) angeordnet ist, am meisten bevorzugt wobei das Befestigungselement ein Befestigungsrahmen ist.

8. Aerosolerzeugungsvorrichtung (10) nach einem beliebigen der vorhergehenden Ansprüche, wobei die Öffnung (14) einen Schlitz aufweist, optional, wobei der Schlitz eine erste und eine zweite Lippe umfasst, die in der flexiblen Membran (12) ausgebildet sind, wobei die erste und zweite Lippe aneinander anliegen.

9. Aerosolerzeugungsvorrichtung (10) nach einem beliebigen der vorhergehenden Ansprüche, wobei der zentrale Abschnitt (14C) eine Öffnung aufweist, wobei die Breite der Öffnung bevorzugt größer ist als die Breite der Schlitze.

10. Bausatz, umfassend eine Aerosolerzeugungsvorrichtung (10) nach einem beliebigen der vorhergehenden Ansprüche und ein Reinigungswerkzeug (20) zum Reinigen des Hohlraums (16) der Aerosolerzeugungsvorrichtung (10).

11. Bausatz nach Anspruch 10, wobei das Reinigungswerkzeug (20) einen Reinigungskopf (20A) aufweist, wobei der Reinigungskopf (20A) zum Öffnen der Öffnung (14) ausgelegt ist, wenn mit dem Reinigungswerkzeug (20) Druck ausgeübt wird, wobei der Reinigungskopf (20A) bevorzugt ausgelegt ist, durch die Öffnung (14) der flexiblen Membran (12) geschoben zu werden.

12. Aerosolerzeugungssystem, umfassend
eine Aerosolerzeugungsvorrichtung (10) nach einem der Ansprüche 1 bis 9,
einen aerosolerzeugenden Artikel zur Aufnahme in dem Hohlraum (16), und
ein Reinigungswerkzeug (20) zum Reinigen des Hohlraums (16).

13. Verfahren zum Reinigen der Aerosolerzeugungsvorrichtung (10) nach einem der Ansprüche 1 bis 9, wobei die Aerosolerzeugungsvorrichtung (10) ein nachgelagertes Ende (10A) zum Aufnehmen des aerosolerzeugenden Artikels in dem Hohlraum (16) umfasst, wobei das Verfahren eines oder beide umfasst von:
- Einführen eines Reinigungswerkzeugs (20) in den Hohlraum (16) durch das nachgelagerte Ende (10A) und Schieben des Reinigungswerkzeugs (20) durch die Öffnung (14) und Entfernen des Reinigungswerkzeugs (20) aus dem Hohlraum (16), oder
- Schieben eines Reinigungswerkzeugs (20) durch die Öffnung (14) der flexiblen Membran (12) in den Hohlraum (16) von dem vorgelagerten Ende (10B) der Aerosolerzeugungsvorrichtung (10); und
Entfernen des Reinigungswerkzeugs (20) aus dem Hohlraum (16).

14. Verfahren zum Reinigen nach dem vorhergehenden Anspruch, wobei das Entfernen des Reinigungswerkzeugs (20) aus dem Hohlraum (16) umfasst:
- Zurückziehen des Reinigungswerkzeugs (20) durch die Öffnung (14) der flexiblen Membran (12) aus dem Hohlraum (16).

## Revendications

1. Dispositif de génération d'aérosol (10) comprenant :
une cavité (16) pour recevoir un article de génération d'aérosol comprenant un substrat formant aérosol, dans lequel le substrat formant aérosol est un substrat solide formant aérosol, et
une membrane souple (12) agencée à l'extrémité amont de la cavité (16), la membrane souple (12) comprenant une ouverture (14) en communication fluidique avec la cavité (16) dans lequel la membrane souple (14) est prévue à l'extrémité amont (10B) du dispositif de génération d'aérosol (10), dans lequel l'ouverture (14) est configurée pour servir d'entrée d'air pour le dispositif de génération d'aérosol (10),
dans lequel au moins deux ouvertures (14) sont formées dans la membrane souple (12), les au moins deux ouvertures comprenant des fentes (14) qui se coupent, et dans lequel les au moins deux fentes entrecroisées (14) forment une portion centrale (14C).

2. Dispositif de génération d'aérosol (10) selon la revendication précédente, dans lequel la cavité (16) a un axe longitudinal central de cavité (16A) et dans lequel l'ouverture (14) est au moins partiellement agencée sur l'axe longitudinal central de cavité (16A).

3. Dispositif de génération d'aérosol (10) selon la revendication précédente, dans lequel le dispositif de génération d'aérosol (10) comprend un axe longitudinal central de dispositif (16C) et dans lequel l'axe longitudinal central de dispositif (10C) est décalé par rapport à l'axe longitudinal central de cavité (16A) .

4. Dispositif de génération d'aérosol (10) selon l'une quelconque des revendications précédentes, dans lequel la membrane souple (12) comprend une partie concave qui se bombe vers l'intérieur de la cavité (16), de préférence dans lequel l'ouverture (14) est formée dans la partie concave de la membrane souple (12).

5. Dispositif de génération d'aérosol (10) selon l'une quelconque des revendications précédentes, dans lequel l'ouverture (14) est configurée pour servir comme la seule entrée d'air pour le dispositif de génération d'aérosol (10).

6. Dispositif de génération d'aérosol (10) selon l'une quelconque des revendications 1 ou 3, dans lequel la membrane souple (12) comprend une partie convexe qui se bombe vers l'extérieur à partir de l'intérieur de la cavité (16), de préférence dans lequel l'ouverture (14) est formée dans la partie convexe de la membrane souple (12).

7. Dispositif de génération d'aérosol (10) selon l'une quelconque des revendications précédentes, comprenant en outre un élément de montage, dans lequel la membrane souple (12) est montée dans l'élément de montage, de préférence dans lequel l'élément de montage est agencé à l'extrémité amont du dispositif de génération d'aérosol (10), de manière davantage préférée dans lequel l'élément de montage est un cadre de montage.

8. Dispositif de génération d'aérosol (10) selon l'une quelconque des revendications précédentes, dans lequel l'ouverture (14) comprend une fente, facultativement dans lequel la fente comprend une première et une deuxième lèvre formées dans la membrane souple (12), les première et deuxième lèvres venant en butée l'une contre l'autre.

9. Dispositif de génération d'aérosol (10) selon l'une quelconque des revendications précédentes, dans lequel la portion centrale (14C) comprend un trou, de préférence dans lequel la largeur du trou est plus grande que la largeur des fentes.

10. Kit comprenant un dispositif de génération d'aérosol (10) selon l'une quelconque des revendications précédentes et un outil de nettoyage (20) pour nettoyer la cavité (16) du dispositif de génération d'aérosol (10).

11. Kit selon la revendication 10, dans lequel l'outil de nettoyage (20) comprend une tête de nettoyage (20A), la tête de nettoyage (20A) étant configurée pour ouvrir l'ouverture (14) lorsqu'une pression est appliquée avec l'outil de nettoyage (20), de préférence dans lequel la tête de nettoyage (20A) est configurée pour être poussée à travers l'ouverture (14) de la membrane souple (12) .

12. Système de génération d'aérosol comprenant
un dispositif de génération d'aérosol (10) selon l'une quelconque des revendications 1 à 9,
un article de génération d'aérosol destiné à être reçu dans la cavité (16), et
un outil de nettoyage (20) pour nettoyer la cavité (16).

13. Procédé de nettoyage du dispositif de génération d'aérosol (10) selon l'une quelconque des revendications 1 à 9, le dispositif de génération d'aérosol (10) comprenant une extrémité aval (10A) pour recevoir l'article de génération d'aérosol dans la cavité (16), le procédé comprenant l'un ou les deux parmi :
- l'insertion d'un outil de nettoyage (20) dans la cavité (16) à travers l'extrémité aval (10A) et la poussée de l'outil de nettoyage (20) à travers l'ouverture (14) et le retrait de l'outil de nettoyage (20) de la cavité (16), ou
- la poussée d'un outil de nettoyage (20) à travers l'ouverture (14) de la membrane souple (12), jusque dans la cavité (16) depuis l'extrémité amont (10B) du dispositif de génération d'aérosol (10) ; et
le retrait de l'outil de nettoyage (20) de la cavité (16).

14. Procédé de nettoyage selon la revendication précédente, dans lequel le retrait de l'outil de nettoyage (20) de la cavité (16) comprend :
- le fait de tirer l'outil de nettoyage (20) à travers l'ouverture (14) de la membrane souple (12) hors de la cavité (16).
